# EUROPEAN PATENT APPLICATION

(11) **EP 3 382 173 A1**
(43) Date of publication of application: **03.10.2018**
(21) Application number: 18163776.0
(22) Date of filing: 23.03.2018
(51) Int. Cl.: F01N 11/00, F02D 41/22, F02D 41/14, G01N 27/417, G01N 33/00

(54) **NOX SENSOR ABNORMALITY DETECTOR**

(30) Priority: 27.03.2017 JP 2017060851
(71) Applicant: Toyota Jidosha Kabushiki Kaisha, Aichi-Ken 471-8571 (JP)
(72) Inventor: NAKAMURA, Yoshitaka, TOYOTA-SHI, AICHI-KEN, 471-8571 (JP); MUTO, Harufumi, TOYOTA-SHI, AICHI-KEN, 471-8571 (JP); ONISHI, Tomomi, TOYOTA-SHI, AICHI-KEN, 471-8571 (JP); HASHIMOTO, Yosuke, TOYOTA-SHI, AICHI-KEN, 471-8571 (JP); TSUJI, Koichi, TOYOTA-SHI, AICHI-KEN, 471-8571 (JP)
(74) Representative: Cabinet Beau de Loménie

(57) **Abstract**

A NOx sensor abnormality detector for a NOx sensor including a measurement chamber, a pump cell, and a sensor cell. The NOx sensor abnormality detector includes a signal receiving section configured to receive the detection value of the sensor cell, a reduction process executing section configured to execute a reduction process that reduces the discharge function of the pump cell, and an abnormality determining section configured to perform abnormality determination of the NOx sensor. The NOx sensor abnormality detector is configured to execute abnormality detection control in which the abnormality determining section performs abnormality determination of the NOx sensor based on the detection value of the sensor cell, which is input to the signal receiving section, after the reduction process executing section starts the reduction process.

## Description

### BACKGROUND

The present invention relates to a NOx sensor abnormality detector.

Japanese Laid-Open Patent Publication No. 2015-1206 discloses an internal combustion engine that includes an exhaust purification device in the exhaust passage to reduce nitrogen oxides (hereinafter referred to as "NOx") in the exhaust gas. The exhaust purification device includes a urea solution supply valve, which is used to supply urea solution into the exhaust gas, and a urea-selective catalytic reduction catalyst (a urea-SCR catalyst), which reduces NOx using the urea solution injected from the urea solution supply valve as the reductant. In the exhaust passage, a first NOx sensor is provided upstream of the exhaust purification device in the exhaust flow, and a second NOx sensor is provided downstream of the exhaust purification device in the exhaust flow. The NOx sensors detect the NOx concentration in the exhaust gas. The amount of urea solution injected from the urea solution supply valve is set according to the values detected by the NOx sensors.

The above-mentioned publication discloses a NOx sensor abnormality detector, which detects abnormality of the second NOx sensor. When the urea-SCR catalyst does not reduce NOx, the NOx concentration in the exhaust gas passing through the second NOx sensor is equivalent to that in the exhaust gas passing through the first NOx sensor. In this abnormality detector, the ammonia adhering to the urea-SCR catalyst is consumed. The abnormality detector detects abnormality based on the output signals from the first and second NOx sensors emitted while the urea solution is not injected from the urea solution supply valve and the urea-SCR catalyst does not reduce NOx.

The NOx sensor abnormality detector detects abnormality of the second NOx sensor based on the output signals of the first and second NOx sensors emitted while the urea-SCR catalyst is not reducing NOx. That is, abnormality detection involves stopping the function of the exhaust purification device provided between the first and second NOx sensors. It is desirable that a NOx sensor abnormality detector be able to detect abnormality regardless of the operation state of the exhaust purification device.

### SUMMARY

In accordance with one aspect, a NOx sensor abnormality detector for a NOx sensor is provided. The NOx sensor includes a measurement chamber, a pump cell, and a sensor cell. The measurement chamber is located in an exhaust passage of an internal combustion engine and into which exhaust gas is introduced. The pump cell has a discharge function of discharging oxygen from the measurement chamber. The sensor cell has a decomposition function of decomposing NOx in the measurement chamber into nitrogen and oxygen and a detection function of detecting an oxygen concentration in the measurement chamber. The NOx sensor is configured to output, as a signal corresponding to a NOx concentration in the exhaust gas, a detection value of oxygen concentration detected by the sensor cell while causing the discharge function of the pump cell to take effect. The NOx sensor abnormality detector includes a signal receiving section configured to receive the detection value of the sensor cell, a reduction process executing section configured to execute a reduction process that reduces the discharge function of the pump cell, and an abnormality determining section configured to perform abnormality determination of the NOx sensor. The NOx sensor abnormality detector is configured to execute abnormality detection control in which the abnormality determining section performs abnormality determination of the NOx sensor based on the detection value of the sensor cell, which is input to the signal receiving section, after the reduction process executing section starts the reduction process.

Exhaust gas includes excess oxygen and NOx after combustion. The NOx sensor detects the NOx concentration in the exhaust gas by detecting, with the sensor cell, the oxygen produced by decomposing the NOx in the exhaust gas, while the excess oxygen in the exhaust gas introduced into the measurement chamber is discharged from the measurement chamber by the pump cell.

In the embodiment described above, the reduction process executing section executes the reduction process to detect an abnormality, so that the discharge function of the pump cell is reduced. Consequently, the excess oxygen in the exhaust gas is not discharged and remains in the measurement chamber. The sensor cell has a function of detecting the oxygen concentration. When the discharge function of the pump cell is reduced, the sensor cell detects the concentration of the sum of the excess oxygen in the measurement chamber and the oxygen produced by decomposition of NOx. However, when an abnormality occurs in the detection function of the sensor cell, the sensor cell fails to detect the oxygen concentration of the sum of the oxygen. Accordingly, when the excess oxygen remains in the measurement chamber after the reduction process starts to reduce the discharge function of the pump cell, the detection value of the sensor cell that operates abnormally and is unable to detect the oxygen concentration differs significantly from the detection value of the sensor cell that operates normally and is able to detect the excess oxygen concentration. The configuration described above uses the characteristics of the NOx sensor to determine an abnormality of the NOx sensor. This achieves abnormality detection regardless of the operation state of the exhaust purification device.

The above-described NOx sensor abnormality detector preferably includes an accumulated value calculating section configured to accumulate detection values of the sensor cell, which are input to the signal receiving section, during a predetermined accumulation period, which starts after the reduction process starts. The abnormality determining section is preferably configured to determine that the NOx sensor operates abnormally when an accumulated value calculated by the accumulated value calculating section is less than or equal to a determination value.

The configuration described above accumulates the detection values of the sensor cell during the predetermined accumulation period, which starts after the reduction process starts, and the abnormality determining section uses the accumulated value to determine whether the NOx sensor operates abnormally. Thus, a temporary reduction in the detection value of the sensor cell caused by some factor while the sensor cell operates normally has limited effect on the abnormality detection. This increases the accuracy of abnormality detection of the NOx sensor.

The above-described NOx sensor abnormality detector preferably includes an idle determining section configured to determine whether the internal combustion engine is at idle. The abnormality determining section is preferably configured to execute the abnormality detection control when the idle determining section determines that the internal combustion engine is at idle.

While the internal combustion engine is at idle and emits exhaust gas at a stable flow rate, the flow rate of the exhaust gas flowing into the measurement chamber is also stable. This limits variations in the detection value of the sensor cell. The configuration described above detects abnormality of the NOx sensor while the detection value of the sensor cell is stable, increasing the accuracy of the abnormality detection, which depends on the detection value of the sensor cell.

Other aspects and advantages of the present invention will become apparent from the following description, taken in conjunction with the accompanying drawings, illustrating by way of example the principles of the invention.

### BRIEF DESCRIPTION OF THE DRAWINGS

The invention, together with objects and advantages thereof, may best be understood by reference to the following description of the presently preferred embodiments together with the accompanying drawings in which:
Fig. 1 is a schematic view showing the structure of a NOx sensor abnormality detector according to a first embodiment;
Fig. 2 is a cross-sectional view schematically showing the structure of the NOx sensor in the Fig. 1;
Fig. 3 is a flowchart showing the flow of process associated with abnormality detection control;
Figs. 4A to 4G are timing charts showing abnormality determination in the abnormality detection control;
Fig. 5 is a functional block diagram of a controller of a NOx sensor abnormality detector according to a second embodiment;
Fig. 6 is a flowchart showing the flow of process associated with abnormality detection control; and
Fig. 7 is a flowchart showing the flow of process associated with abnormality detection control of a NOx sensor abnormality detector according to a third embodiment.

### DESCRIPTION OF THE PREFERRED EMBODIMENTS

### First Embodiment

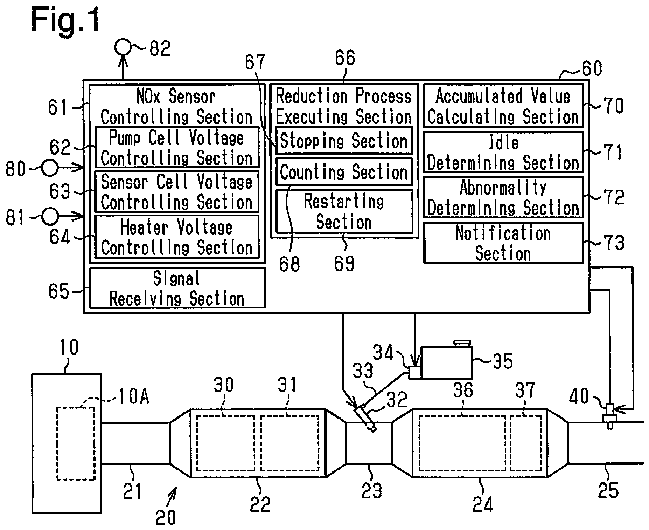

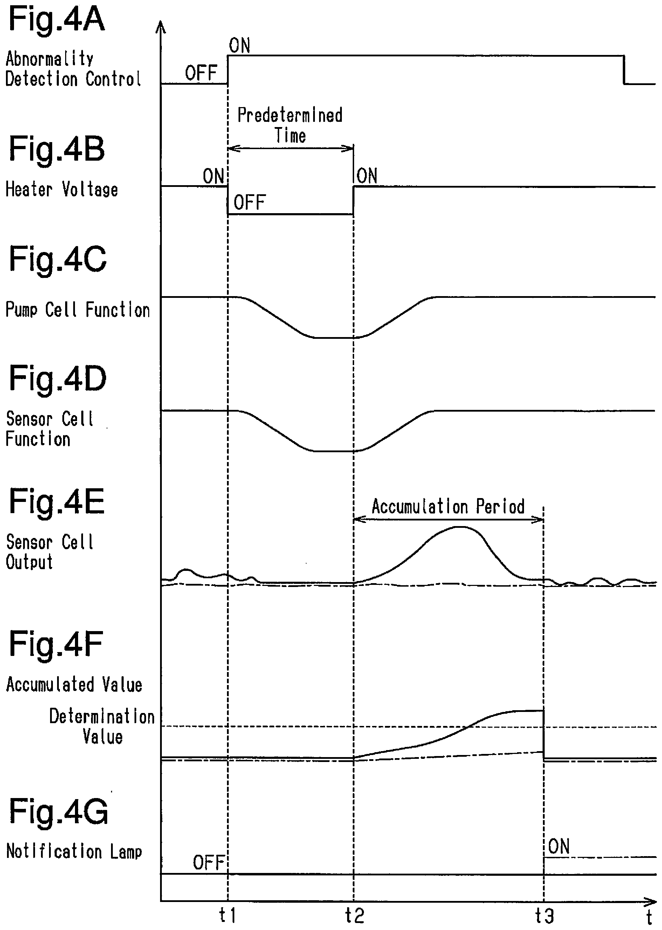

Referring to Figs. 1 to 4G, a NOx sensor abnormality detector according to a first embodiment is now described. The present embodiment is used for a NOx sensor placed in the exhaust passage of a diesel engine serving as an internal combustion engine.

As shown in Fig. 1, the engine body 10 of the internal combustion engine includes a combustion chamber 10A. The engine body 10 is coupled to an exhaust passage 20, into which exhaust gas is discharged from the combustion chamber 10A. The exhaust passage 20 includes a first exhaust pipe 21, which is coupled to the engine body 10, and a first catalytic converter 22, which is coupled to the first exhaust pipe 21. The first catalytic converter 22 includes an oxidation catalyst 30 and a PM filter 31 arranged inside the first catalytic converter 22. The oxidation catalyst 30 is closer to the first exhaust pipe 21, that is, on the left side as viewed in Fig. 1.

The oxidation catalyst 30 oxidizes hydrocarbons (HC) and carbon monoxide (CO) in the exhaust gas and produces water and carbon dioxide. The PM filter 31 traps particulate matter (PM) in the exhaust gas. The end of the first catalytic converter 22 on the downstream side in the exhaust flow is coupled to a second exhaust pipe 23. The second exhaust pipe 23 includes a urea solution supply valve 32 for supplying a urea solution into the exhaust gas. The urea solution supply valve 32 is coupled to one end of a urea solution supply passage 33. The other end of the urea solution supply passage 33 is coupled to a urea solution pump 34, which is coupled to a urea solution tank 35. The urea solution stored in the urea solution tank 35 is pumped out by the urea solution pump 34 and supplied to the urea solution supply valve 32 through the urea solution supply passage 33. A second catalytic converter 24 is coupled to the end of the second exhaust pipe 23 on the downstream side in the exhaust flow. The second catalytic converter 24 includes a urea-SCR catalyst 36 and an ammonia slip catalyst 37. The urea-SCR catalyst 36 is closer to the second exhaust pipe 23, that is, on the left side as viewed in Fig. 1.

The urea-SCR catalyst 36 reduces NOx in the exhaust gas using the urea solution injected from the urea solution supply valve 32 as the reductant. That is, the urea solution injected from the urea solution supply valve 32 is thermally decomposed into ammonia. The urea-SCR catalyst 36 uses the ammonia to reduce NOx into nitrogen and water. When an excessive amount of urea solution with respect to the NOx in the exhaust gas is injected from the urea solution supply valve 32, ammonia may pass through the urea-SCR catalyst 36 and flow downstream in the exhaust flow, that is, flow to the ammonia slip catalyst 37. The ammonia slip catalyst 37 oxidizes the ammonia that has passed through the urea-SCR catalyst 36 and produces nitrogen and water. The end of the second catalytic converter 24 on the downstream side in the exhaust flow is coupled to a third exhaust pipe 25. The third exhaust pipe 25 includes a NOx sensor 40 for detecting the NOx concentration in the exhaust gas.

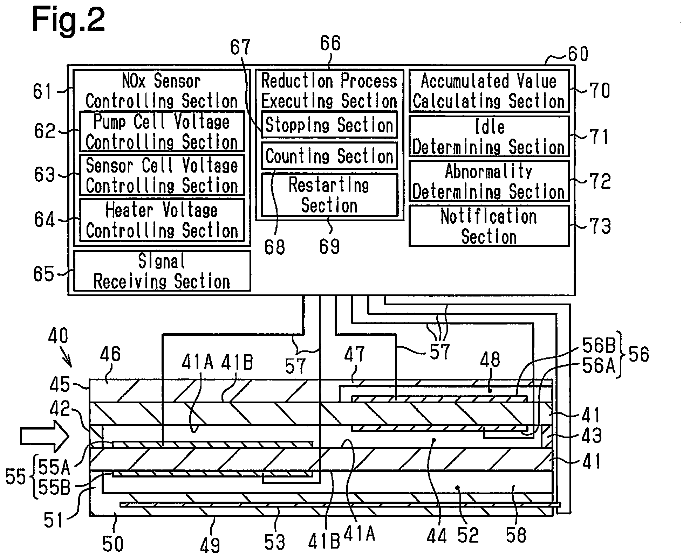

As shown in Fig. 2, the NOx sensor 40 includes two solid electrolytes 41, which face each other. The solid electrolytes 41 are planar and include facing surfaces 41A, which face each other. The solid electrolytes 41 are made of an oxygen ion conductive solid electrolyte material, such as zirconium dioxide. A diffusion resistive element 42 is placed at the first end (the left end as viewed in Fig. 2) of the pair of solid electrolytes 41 and sandwiched by the two facing surfaces 41A. The diffusion resistive element 42 is made of a porous material, for example, and limits the flow velocity of the passing exhaust gas. A wall 43 is placed at the second end (the right end as viewed in Fig. 2) of the pair of solid electrolytes 41 and sandwiched by the two facing surfaces 41A. The wall 43 is made of an insulator, such as alumina.

One of the two solid electrolytes 41 (the upper solid electrolyte 41 as viewed in Fig. 2) is coupled to a first holder 45, which is made of an insulator, such as alumina. The first holder 45 includes a contact section 46, which is coupled to the back surface 41B (the upper surface as viewed in Fig. 2) of the solid electrolyte 41, which is opposite to the facing surface 41A. The contact section 46 extends from the first end (the left end as viewed in Fig. 2) to the second end (the right end as viewed in Fig. 2) of the solid electrolyte 41. The contact section 46 is coupled to a separation section 47, which is separated from the solid electrolyte 41. The separation section 47 extends from the contact section 46 to the position corresponding to the second end of the solid electrolyte 41.

The other of the two solid electrolytes 41 (the lower solid electrolyte 41 as viewed in Fig. 2) is coupled to a second holder 49, which is made of an insulator, such as alumina. The second holder 49 includes a facing section 50, which faces the back surface 41B (the lower surface as viewed in Fig. 2) of the solid electrolyte 41, which is opposite to the facing surface 41A. The facing section 50 is separated from the solid electrolyte 41. One end (the left end as viewed in Fig. 2) of the facing section 50 is coupled to a coupling section 51, which is extended toward and coupled to the solid electrolyte 41. The second holder 49 includes a heater 53 embedded in the facing section 50. The amount of heat generated by the heater 53 varies depending on the applied voltage.

The NOx sensor 40 includes a pair of side walls 58 sandwiching the pair of solid electrolytes 41, the diffusion resistive element 42, the wall 43, the first holder 45, and the second holder 49 in a predetermined direction (the depth direction of Fig. 2). The ends in the predetermined direction of each of the pair of solid electrolytes 41, the diffusion resistive element 42, the wall 43, the first holder 45, and the second holder 49 are coupled to the side walls 58.

As such, the NOx sensor 40 includes a measurement chamber 44, which is defined by the pair of solid electrolytes 41, the diffusion resistive element 42, the wall 43, and the pair of side walls 58. In addition, the first holder 45, one of the solid electrolytes 41, and the pair of side walls 58 define a first discharge chamber 48, which is open at the second end of the solid electrolytes 41 (at the right end as viewed in Fig. 2). Further, the second holder 49, the other solid electrolyte 41, and the pair of side walls 58 define a second discharge chamber 52, which is open at the second end of the solid electrolytes 41 (at the right end as viewed in Fig. 2). As indicated by the arrow in Fig. 2, exhaust gas is introduced into the measurement chamber 44 through the diffusion resistive element 42.

The NOx sensor 40 includes a pump cell 55, which has a discharge function of discharging oxygen from the measurement chamber 44 to the second discharge chamber 52. The pump cell 55 is placed on the side where the diffusion resistive element 42 is located (the left side as viewed in Fig. 2). The pump cell 55 includes a pump cathode electrode 55A arranged in the measurement chamber 44 and a pump anode electrode 55B arranged in the second discharge chamber 52. The pump cathode electrode 55A and the pump anode electrode 55B are made of a noble metal that does not reduce NOx, such as an Au-Pt alloy (a gold-platinum alloy). The pump cathode electrode 55A is fixed to the facing surface 41A of the lower solid electrolyte 41, and the pump anode electrode 55B is fixed to the back surface 41B of the lower solid electrolyte 41.

While voltage is applied between the pump cathode electrode 55A and the pump anode electrode 55B of the pump cell 55, the oxygen in exhaust gas in the measurement chamber 44 becomes oxygen ion upon contact with the pump cathode electrode 55A. The oxygen ion moves from the pump cathode electrode 55A to the pump anode electrode 55B through the solid electrolyte 41, becomes oxygen when releasing electric charge at the pump anode electrode 55B, and is discharged into the second discharge chamber 52. The discharged oxygen returns to the exhaust gas through the opening of the second discharge chamber 52. The pump cell 55 thus has the discharge function of discharging oxygen from the measurement chamber 44 when voltage is applied between the pump cathode electrode 55A and the pump anode electrode 55B. The higher the voltage applied between the pump cathode electrode 55A and the pump anode electrode 55B of the pump cell 55, the more the amount per unit time of the oxygen discharged from the measurement chamber 44.

The NOx sensor 40 also includes a sensor cell 56, which has a decomposition function of decomposing the NOx in the measurement chamber 44 into nitrogen and oxygen and a detection function of detecting the oxygen concentration in the measurement chamber 44. The sensor cell 56 is placed on the side where the wall 43, which is opposite to the diffusion resistive element 42, is located (the right side as viewed in Fig. 2). The sensor cell 56 includes a sensor cathode electrode 56A arranged in the measurement chamber 44 and a sensor anode electrode 56B arranged in the first discharge chamber 48. The sensor cathode electrode 56A is fixed to the facing surface 41A of the upper solid electrolyte 41, and the sensor anode electrode 56B is fixed to the back surface 41B of the upper solid electrolyte 41. The sensor cathode electrode 56A and the sensor anode electrode 56B are made of a noble metal that has a high reduction effect on NOx, such as a Pt-Rh alloy (a platinum-rhodium alloy). As such, when coming into contact with the sensor cathode electrode 56A, the NOx in exhaust gas in the measurement chamber 44 is reduced and decomposed into nitrogen and oxygen.

While voltage is applied between the sensor cathode electrode 56A and the sensor anode electrode 56B of the sensor cell 56, the oxygen in exhaust gas in the measurement chamber 44 becomes oxygen ion upon contact with the sensor cathode electrode 56A. The oxygen ion moves from the sensor cathode electrode 56A to the sensor anode electrode 56B through the solid electrolyte 41, becomes oxygen when releasing electric charge at the sensor anode electrode 56B, and is discharged into the first discharge chamber 48. The discharged oxygen returns to the exhaust gas through the opening of the first discharge chamber 48. While voltage is applied between the sensor cathode electrode 56A and the sensor anode electrode 56B, the sensor cell 56 outputs, as detection values, the current generated when the oxygen ion moves from the sensor cathode electrode 56A to the sensor anode electrode 56B. The value of current is higher when a greater amount of oxygen ion moves. That is, the sensor cell 56 has a detection function of detecting the concentration of the accumulated value of the oxygen generated by the reduction of NOx and the oxygen contained in the exhaust gas introduced into the measurement chamber 44.

In the NOx sensor 40, voltage is applied to the heater 53, allowing the heater 53 to generate heat, which is transmitted through the pair of side walls 58 and the pair of solid electrolytes 41 to heat the pump cell 55 and the sensor cell 56. Voltage is also applied to the pump cell 55 and the sensor cell 56. The application of voltage heats the pump cell 55 and the sensor cell 56 to a predetermined activation temperature, allowing the cells 55, 56 to function. Exhaust gas contains excess oxygen and NOx after combustion. In the NOx sensor 40, the pump cell 55 discharges the excess oxygen, which is contained in the exhaust gas introduced into the measurement chamber 44, from the measurement chamber 44, and the sensor cell 56 discharges the oxygen produced by decomposing NOx in the exhaust gas. The NOx sensor 40 outputs the value of current generated when the sensor cell 56 discharges the oxygen, that is, the detection value of oxygen concentration, as a signal corresponding to the NOx concentration in the exhaust gas.

As shown in Fig. 1, the output signal from the NOx sensor 40 is input to the controller 60 of the internal combustion engine. As shown in Fig. 2, the cells 55, 56 and the heater 53 of the NOx sensor 40 are connected to the controller 60 through wires 57. The controller 60 applies voltage to the cells 55, 56 and the heater 53 and controls the operation amount of the urea solution pump 34 and the valve opening period of the urea solution supply valve 32 based on the output signal from the NOx sensor 40. That is, in order to minimize the NOx concentration detected by the NOx sensor 40, the controller 60 adjusts the amount of urea solution supplied to the exhaust gas to control the NOx concentration in the exhaust gas in the third exhaust pipe 25.

As shown in Figs. 1 and 2, the controller 60 includes as functional parts a NOx sensor controlling section 61, a signal receiving section 65, a reduction process executing section 66, an accumulated value calculating section 70, an idle determining section 71, an abnormality determining section 72, and a notification section 73. The controller 60 is configured to execute abnormality detection control for detecting abnormality of the NOx sensor 40. In addition, the controller 60 receives output signals from an engine speed sensor 80, which detects the engine speed of the internal combustion engine, and an acceleration sensor 81, which detects the depression amount of the accelerator pedal.

The NOx sensor controlling section 61 includes a pump cell voltage controlling section 62, which controls the voltage applied to the pump cell 55, a sensor cell voltage controlling section 63, which controls the voltage applied to the sensor cell 56, and a heater voltage controlling section 64, which controls the voltage applied to the heater 53.

The detection value of the sensor cell 56 is input to the signal receiving section 65. The reduction process executing section 66 is configured to execute a reduction process that reduces the discharge function of the pump cell 55. The reduction process executing section 66 includes a stopping section 67, a counting section 68, and a restarting section 69. The stopping section 67 is configured to start the reduction process by controlling the heater voltage controlling section 64 to stop the application of voltage to the heater 53 of the NOx sensor 40. Starting the reduction process stops the heating of the pump cell 55, and the reduced temperature of the pump cell 55 reduces the discharge function. The counting section 68 is configured to measure the time elapsed since the stopping section 67 stops the application of voltage to the heater 53. When the time measured by the counting section 68 reaches a predetermined time, the restarting section 69 controls the heater voltage controlling section 64 to restart the application of voltage to the heater 53, thereby ending the reduction process. This restarts heating of the pump cell 55, allowing the temperature of the pump cell 55 to be maintained at the predetermined activation temperature or higher.

The accumulated value calculating section 70 is configured to accumulate the detection values of the sensor cell 56, which are input to the signal receiving section 65, during a predetermined accumulation period, which starts after the reduction process executing section 66 starts the reduction process. The accumulated value calculating section 70 of the present embodiment starts addition of the detection values of the sensor cell 56 when the reduction process executing section 66 stops the reduction process. The idle determining section 71 is configured to determine whether the internal combustion engine is at idle based on output signals from the engine speed sensor 80 and the acceleration sensor 81. The abnormality determining section 72 is configured to determine that the NOx sensor 40 operates abnormally when the accumulated value calculated by the accumulated value calculating section 70 is less than or equal to a determination value.

As shown in Fig. 1, the abnormality detector for the NOx sensor 40 also includes a notification lamp 82. The notification section 73 is configured to turn on the notification lamp 82 to make a notification about the abnormality when the abnormality determining section 72 determines that the NOx sensor 40 operates abnormally. The abnormality detector for the NOx sensor 40 includes the controller 60, the engine speed sensor 80, the acceleration sensor 81, and the notification lamp 82.

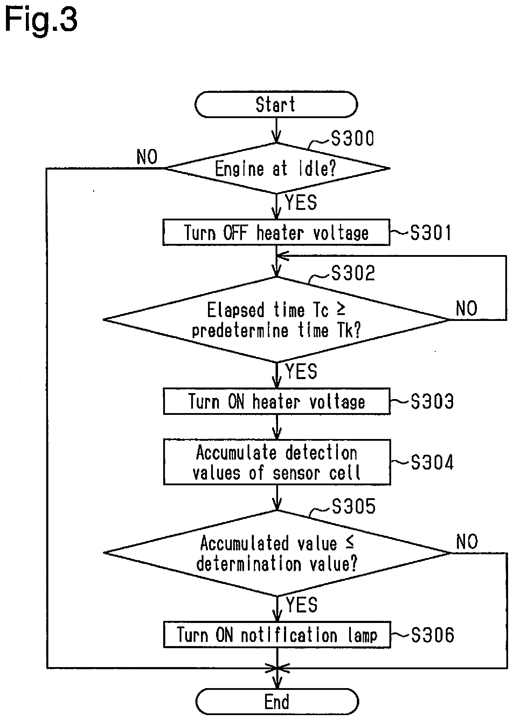

Referring to the flowchart of Fig. 3, the flow of process associated with the abnormality detection control performed by the controller 60 is now described. This process is repeated at predetermined intervals.

Fig. 3 shows how the controller 60 performs the process. Firstly, the idle determining section 71 determines whether the internal combustion engine is at idle (step S300). If the internal combustion engine is at idle (step S300: YES), the controller 60 proceeds to step S301 and performs abnormality detection control. In the abnormality detection control, the reduction process executing section 66 first executes the reduction process. That is, at step S301, the stopping section 67 starts the reduction process by controlling the heater voltage controlling section 64 to stop the application of voltage to the heater 53 of the NOx sensor 40. Stopping the application of voltage to the heater 53 lowers the temperatures of the pump cell 55 and the sensor cell 56 of the NOx sensor 40, causing the functions of the pump cell 55 and the sensor cell 56 to gradually decrease. The counting section 68 measures the time elapsed since the application of voltage to the heater 53 is stopped.

Then, the controller 60 proceeds to step S302 and determines whether the elapsed time Tc measured by the counting section 68 has reached the predetermined time Tk. If step S302 determines that the elapsed time Tc has not reached the predetermined time Tk (step S302: NO), the controller 60 does not proceed to the subsequent process and repeats step S302. When the elapsed time Tc reaches the predetermined time Tk after some time, the determination at step S302 becomes affirmative (step S302: YES), allowing the restarting section 69 to control the heater voltage controlling section 64 to restart the application of voltage to the heater 53 (step S303). As the predetermined time Tk, a time is set in which the discharge function of the pump cell 55 is reduced and such a state continues for a specific time. Thus, when the elapsed time Tc reaches the predetermined time Tk, the oxygen concentration in the measurement chamber 44 has risen to a concentration substantially equal to that in the exhaust gas.

After the reduction process is performed, the accumulated value calculating section 70 adds the detection values of the sensor cell 56, which are input to the signal receiving section 65, during the accumulation period, which starts when the reduction process executing section 66 ends the reduction process, that is, when the restarting section 69 restarts the application of voltage to the heater 53 (step S304). The present embodiment sets as the accumulation period a period that is required for the discharge function of the pump cell 55 to take effect after the application of voltage to the heater 53 restarts so that the excess oxygen introduced in the measurement chamber 44 is substantially entirely discharged to the second discharge chamber 52, and that is required for the detection function of the sensor cell 56 to continue to take effect for a specific time. This accumulation period may also be determined in advance by experiment, as with the predetermined time Tk.

Then, the controller 60 proceeds to step S305, where the abnormality determining section 72 determines whether the calculated accumulated value is less than or equal to the determination value. When the reduction process executing section 66 performs the reduction process and the discharge function of the pump cell 55 is reduced, the excess oxygen in the exhaust gas is not discharged and remains in the measurement chamber 44. After the discharge function of the pump cell 55 is reduced, the sensor cell 56, which has the detection function of detecting the oxygen concentration, detects the concentration of the accumulated value of the excess oxygen in the measurement chamber 44 and the oxygen produced by decomposition of NOx. Therefore, the accumulated value will be large when the detection function of the sensor cell 56 is normal. In contrast, when abnormality occurs in the detection function of the sensor cell 56, the sensor cell 56 fails to detect the oxygen concentration in the measurement chamber 44. This results in a small accumulated value.

The present embodiment sets as the determination value the maximum value of accumulated value that is expected when the sensor cell 56 operates abnormally, for example. The determination value may be obtained in advance by experiment. If the determination at step S305 is affirmative (step S305: YES), that is, if the accumulated value is less than or equal to the determination value, the controller 60 determines that the NOx sensor 40 operates abnormally and proceeds to step S306, where the notification section 73 turns on the notification lamp 82. The controller 60 then ends the process associated with the abnormality detection control.

If the determination at step S305 is negative (step S305: NO), that is, if the accumulated value exceeds the determination value, the controller 60 determines that the NOx sensor 40 is normal and ends the process associated with the abnormality detection control without proceeding to step S306.

If the controller 60 determines at step S300 that the internal combustion engine is not at idle (step S300: NO), the controller 60 ends the process associated with the abnormality detection control without performing the subsequent process.

Referring to the timing charts of Figs. 4A to 4G, abnormality determination in the abnormality detection control is now described.

As shown in Fig. 4A, the controller 60 executes the abnormality detection control if the internal combustion engine is at idle at the execution timing of the process associated with the abnormality detection control (time point t1). When the abnormality detection control is executed, the reduction process stops the application of voltage to the heater 53 as shown in Fig. 4B. As shown in Figs. 4C and 4D, stopping the application of voltage to the heater 53 gradually reduces the functions of the pump cell 55 and the sensor cell 56.

As indicated by the solid line in Fig. 4E, before starting the abnormality detection control (before the time point t1), the detection values of the sensor cell 56 correspond to the detected NOx concentration in the exhaust gas. While the internal combustion engine is running, the reduction of NOx by the urea-SCR catalyst 36 maintains the NOx concentration in the exhaust gas low. Accordingly, the difference is small between the detection values obtained while the sensor cell 56 operates normally, which are indicated by the solid line in Fig. 4E, and the detection values obtained while the sensor cell 56 operates abnormally, which are indicated by the long dashed short dashed line in Fig. 4E. When the reduction process starts at the time point t1, the function of the sensor cell 56 decreases, narrowing the difference between the detection values obtained while the sensor cell 56 operates normally and the detection values obtained while the sensor cell 56 operates abnormally.

Then, as shown in Fig. 4B, the application of voltage to the heater 53 restarts at a time point t2 at which the predetermined time Tk has elapsed since stopping the application of voltage to the heater 53, that is, the time point t2 at which the function of the pump cell 55 has been reduced by stopping the application of voltage to the heater 53 and such a state has continued for a specific time. Consequently, the functions of the pump cell 55 and the sensor cell 56 gradually recover as shown in Figs. 4C and 4D. Since the excess oxygen still remains in the measurement chamber 44 of the NOx sensor 40 in this state, the sensor cell 56 that operates normally detects, as its detection function recovers, the concentration of the accumulated value of the excess oxygen in the measurement chamber 44 and the oxygen produced by decomposition of NOx. This results in an increase in detection values as indicated by the solid line in Fig. 4E. As indicated by the solid line in Fig. 4F, the accumulated value of the detection values of the sensor cell 56 increases accordingly after the time point t2. Thus, at a time point t3, at which the accumulation period, in which the detection values of the sensor cell 56 are accumulated, has elapsed, the accumulated value exceeds the determination value if the sensor cell 56 is normal. Consequently, as indicated by the solid line in Fig. 4G, the notification lamp 82 is not turned on at the time point t3.

In contrast, as indicated by the long dashed short dashed line in Fig. 4E, the sensor cell 56 that operates abnormally fails to adequately detect the oxygen concentration, although the excess oxygen still remains in the measurement chamber 44 of the NOx sensor 40 after the time point t2. Thus, unlike when the sensor cell 56 operates normally, the detection values of the sensor cell 56 do not increase. As indicated by the long dashed short dashed line in Fig. 4F, the accumulated value of detection values of the sensor cell 56 increases slowly after the time point t2. Thus, at the time point t3, at which the accumulation period, in which the detection values of the sensor cell 56 are accumulated, has elapsed, the accumulated value is less than or equal to the determination value If the sensor cell 56 operates abnormally. If the accumulated value of the detection values of the sensor cell 56 is determined to be less than or equal to the determination value at the time point t3, the notification lamp 82 is turned on as indicated by the long dashed short dashed line in Fig. 4G.

The present embodiment achieves the following advantages.
(1) In the present embodiment, in order to detect an abnormality, the reduction process executing section 66 performs the reduction process so that the discharge function of the pump cell 55 is reduced. Consequently, the excess oxygen in the exhaust gas is not discharged and remains in the measurement chamber 44. When the excess oxygen remains in the measurement chamber 44, the detection values of the sensor cell 56 that operates abnormally and is unable to detect the oxygen concentration differ significantly from the detection values of the sensor cell 56 that operates normally and able to detect the oxygen concentration. As such, the use of the characteristics of the NOx sensor 40 allows for abnormality determination of the NOx sensor 40 regardless of the operation state of the exhaust purification device, such as the urea solution supply valve 32 or the urea-SCR catalyst 36.
(2) The present embodiment accumulates the detection values of the sensor cell 56 during the predetermined accumulation period after starting the reduction process, and uses the accumulated value to determine whether the NOx sensor 40 operates abnormally. Thus, a temporary reduction in the detection values of the sensor cell 56 caused by some factor while the sensor cell 56 operates normally has limited effect on the abnormality detection. This increases the accuracy of abnormality detection of the NOx sensor 40.
(3) While the internal combustion engine is at idle and emits exhaust gas at a stable flow rate, the flow rate of the exhaust gas flowing into the measurement chamber 44 is also stable. This limits variations in the detection values of the sensor cell 56. In the present embodiment, the abnormality detection control is performed when the internal combustion engine is determined to be at idle, so that the abnormality detection of the NOx sensor 40 is performed when the detection values of the sensor cell 56 are stable. This helps to increase the accuracy of the abnormality detection, which depends on the detection values of the sensor cell 56.

Further, during idling, the NOx concentration in the exhaust gas is stable. Thus, although the function of the sensor cell 56 is reduced by the abnormality detection control of the NOx sensor 40, the NOx concentration detected by the NOx sensor 40 before starting the abnormality detection control may be used to control the urea solution supply valve 32 and the urea solution pump 34, enabling proper reduction of the NOx contained in the exhaust gas.

### Second Embodiment

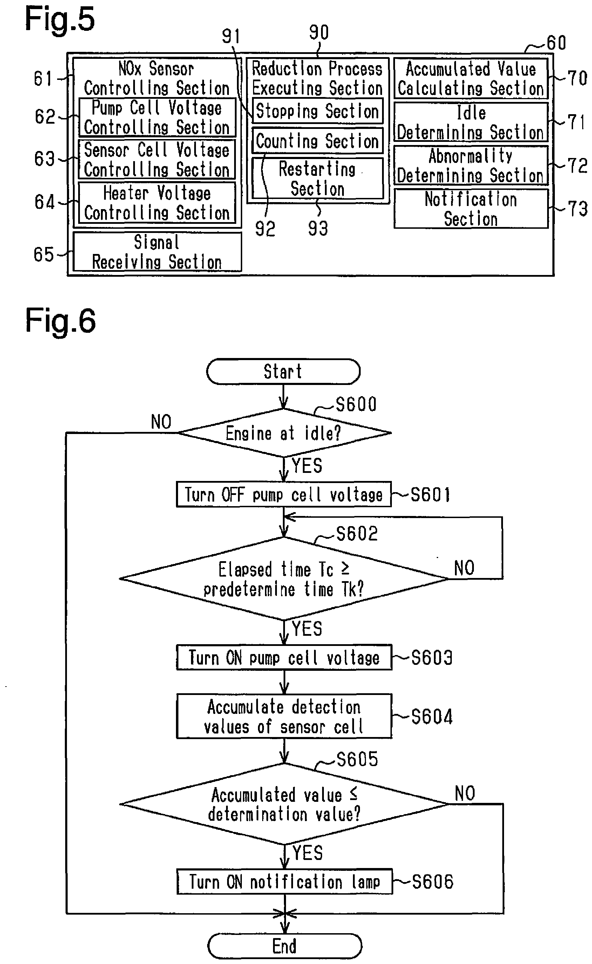

Referring to Figs. 5 and 6, a NOx sensor abnormality detector according to a second embodiment is now described. This embodiment differs from the first embodiment in the reduction process executed by the reduction process executing section in the abnormality detection control performed by the NOx sensor abnormality detector. Same reference numerals are given to those components that are the same as the corresponding components of the first embodiment. Such components will not be described in detail.

As shown in Fig. 5, a controller 60 includes as functional parts a NOx sensor controlling section 61, a signal receiving section 65, a reduction process executing section 90, an accumulated value calculating section 70, an idle determining section 71, an abnormality determining section 72, and a notification section 73.

The reduction process executing section 90 is configured to execute a reduction process that reduces the discharge function of the pump cell 55. The reduction process executing section 90 includes a stopping section 91, a counting section 92, and a restarting section 93. The stopping section 91 is configured to control the pump cell voltage controlling section 62 to stop the application of voltage to the pump cell 55. The counting section 92 is configured to measure the time elapsed since the stopping section 91 stops the application of voltage to the pump cell 55. The restarting section 93 is configured to control the pump cell voltage controlling section 62 to restart the application of voltage to the pump cell 55 when the time measured by the counting section 92 reaches a predetermined time.

Referring to the flowchart of Fig. 6, the flow of process associated with the abnormality detection control performed by the controller 60 is now described. This process is repeated at predetermined intervals.

Fig. 6 shows how the controller 60 performs the process. Firstly, the idle determining section 71 determines whether the internal combustion engine is at idle (step S600). If the internal combustion engine is at idle (step S600: YES), the controller 60 proceeds to step S601 and performs abnormality detection control. In the abnormality detection control, the reduction process executing section 90 first performs the reduction process. That is, at step S601, the stopping section 91 starts the reduction process by controlling the pump cell voltage controlling section 62 to stop the application of voltage to the pump cell 55 of the NOx sensor 40. Stopping the application of voltage to the pump cell 55 stops the function of the pump cell 55 while maintaining the function of the sensor cell 56 of the NOx sensor 40. The counting section 92 measures the time elapsed since stopping the application of voltage to the pump cell 55.

Then, the controller 60 proceeds to step S602 and determines whether the elapsed time Tc measured by the counting section 68 has reached the predetermined time Tk. If step S602 determines that the elapsed time Tc has not reached the predetermined time Tk (step S602: NO), the controller 60 does not proceed to the subsequent process and repeats step S602. When the elapsed time Tc reaches the predetermined time Tk after some time, the determination at S602 becomes positive (step S602: YES), allowing the restarting section 93 to control the pump cell voltage controlling section 62 to restart the application of voltage to the pump cell 55 (step S603). As the predetermined time Tk, a time is set in which the discharge function of the pump cell 55 is stopped and such a state continues for a specific time. When the elapsed time Tc reaches the predetermined time Tk, the oxygen concentration in the measurement chamber 44 has risen to a concentration substantially equal to that in the exhaust gas.

After the reduction process is performed, the accumulated value calculating section 70 accumulates the detection values of the sensor cell 56, which are input to the signal receiving section 65, during a predetermined accumulation period, which starts when the reduction process executing section 66 ends the reduction process, that is, when the restarting section 93 restarts the application of voltage to the pump cell 55 (step S604). The present embodiment sets as the accumulation period a period that is required for the discharge function of the pump cell 55 to take effect after the application of voltage to the pump cell 55 starts so that the excess oxygen in the exhaust gas introduced into the measurement chamber 44 is substantially entirely discharged to the second discharge chamber 52. This accumulation period may also be determined in advance by experiment, as with the predetermined time Tk.

Then, the controller 60 proceeds to step S605, where the abnormality determining section 72 determines whether the calculated accumulated value is less than or equal to the determination value. If step S605 determines that the accumulated value is less than or equal to the determination value (step S605: YES), the controller 60 proceeds to step S606, where the notification section 73 turns on the notification lamp 82. The controller 60 then ends the process associated with the abnormality detection control. If step S605 determines that the accumulated value exceeds the determination value (step S605: NO), the controller 60 ends the process associated with the abnormality detection control without proceeding to step S606.

If step S600 determines that the internal combustion engine is not at idle (step S600: NO), the controller 60 ends the process associated with the abnormality detection control without performing the subsequent process.

In addition to the advantages (1) to (3) of the first embodiment, the second embodiment achieves the following advantage.
(4) The reduction process executing section 90 performs the reduction process by controlling the pump cell voltage controlling section 62 to reduce the function of the pump cell 55. Unlike the first embodiment, in which the discharge function of the pump cell 55 is reduced by stopping the application of voltage to the heater 53 to reduce the temperature of the pump cell 55, the discharge function of the pump cell 55 is reduced directly. This reduces the discharge function of the pump cell 55 more reliably.

### Third Embodiment

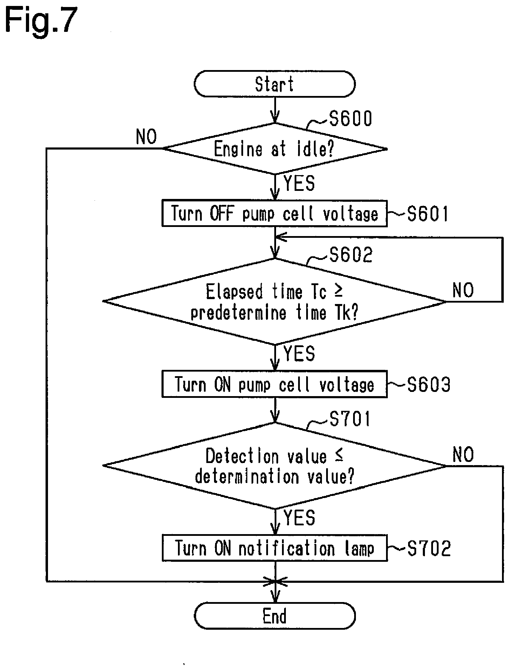

Referring to Fig. 7, a NOx sensor abnormality detector according to a third embodiment is now described. The present embodiment differs from the second embodiment in the method for determining abnormality of a NOx sensor performed by the abnormality determining section in the abnormality detection control performed by the NOx sensor abnormality detector. Same reference numerals are given to those components that are the same as the corresponding components of the first embodiment. Such components will not be described in detail. In the flowchart shown in Fig. 7, the process from step S600 to step S603 is the same as that in the flowchart of Fig. 6 and therefore not described.

As shown in Fig. 7, after the restarting section 93 restarts the application of voltage to the pump cell 55 by controlling the pump cell voltage controlling section 62 at step S603, the controller 60 proceeds to step S701. At step S701, the controller 60 determines whether the detection value of the sensor cell 56, which is input to the signal receiving section 65, is less than or equal to a determination value. As described above, the process from step S601 to step S603 performs the reduction process causing the excess oxygen contained in the exhaust gas to remain in the measurement chamber 44, while the function of the sensor cell 56 of the NOx sensor 40 is maintained. Accordingly, the detection values of the sensor cell 56 are high when the detection function of the sensor cell 56 is normal, whereas the detection values of the sensor cell 56 are low when the detection function of the sensor cell 56 is abnormal. The present embodiment sets as the determination value the maximum value of detection value that is expected when the sensor cell 56 operates abnormally, for example. The determination value may be obtained in advance by experiment.

If the determination at step S701 is affirmative (step S701: YES), that is, if the detection value is less than or equal to the determination value, the controller 60 determines that the NOx sensor 40 operates abnormally and proceeds to step S702. At step S702, the notification section 73 turns on the notification lamp 82. The controller 60 then ends the process associated with the abnormality detection control.

If the determination at step S701 is negative (step S701: NO), that is, if the detection value exceeds the determination value, the controller 60 determines that the NOx sensor 40 operates normally and ends the process associated with the abnormality detection control without proceeding to step S702. In this structure, after the reduction process executing section 90 starts the reduction process, the abnormality determining section 72 compares the detection value of the sensor cell 56, which is input to the signal receiving section 65, with the determination value to perform abnormality determination of the NOx sensor 40. Thus, the structure still has the advantages (1) and (3).

The above-described embodiments may be modified as follows. The following modifications may be combined as necessary.

In the first embodiment, after the reduction process executing section 66 starts the reduction process, the abnormality determining section 72 may compare the detection value of the sensor cell 56, which is input to the signal receiving section 65, with the determination value to perform abnormality determination of the NOx sensor 40, in a similar manner as the third embodiment. In this case, after ending the reduction process, abnormality determination may be performed using the detection value obtained after the detection function of the sensor cell 56 is recovered. In such a configuration, after the restarting section 69 controls the heater voltage controlling section 64 to restart the application of voltage to the heater 53, detection values may be stored for a predetermined period, and the largest value among the stored detection values may be compared with the determination value to determine abnormality.

In the second and third embodiments, the application of voltage to the pump cell 55 is restarted after the predetermined time Tk has elapsed since the reduction process executing section 90 starts the reduction process and stops the application of voltage to the pump cell 55. However, the timing of restarting the application of voltage to the pump cell 55 may be changed. For example, the application of voltage to the pump cell 55 may be restarted when the process associated with the abnormality detection control performed by the controller 60 ends. In this case, step S603 in the flowcharts of Figs. 6 and 7 may be omitted, and the reduction process ends when the process associated with the abnormality detection control ends.

In each of the embodiments described above, the NOx sensor abnormality detector performs abnormality detection control when the internal combustion engine is at idle. Instead, the abnormality detection control may be performed irrespective of whether the engine is at idle. In this case, step S300 in the flowchart of Fig. 3 and step S600 in the flowcharts of Figs. 6 and 7 may be omitted. Such a configuration performs the abnormality detection control at predetermined intervals at which the process associated with the abnormality detection control is performed.

In each of the embodiments, the period required for the oxygen concentration in the measurement chamber 44 to increase to substantially the same concentration in the exhaust gas is set as the predetermined time Tk for stopping the application of voltage to the heater 53. However, the predetermined time Tk may be modified. That is, the predetermined time Tk may be any period that allows a significant difference to occur between a normal sensor cell 56 and an abnormal sensor cell 56 in the accumulated value calculated by the accumulated value calculating section 70 and in the detection values of the sensor cell 56 obtained after the oxygen concentration in the measurement chamber 44 has increased to a suitable level.

The predetermined time Tk may be set in advance or set each time the abnormality detection control is performed. The predetermined time Tk may be set in advance based on experiment. When the predetermined time Tk is variable, the NOx sensor 40 may include a temperature sensor that measures the temperature of the pump cell 55. The time required for the discharge function of the pump cell 55 to decrease may be calculated based on the parameters such as the temperature of the pump cell 55 and the temperature of the exhaust gas measured when stopping the application of voltage to the heater 53, and the predetermined time Tk may be the time obtained by adding a specific time to the calculated time.

Alternatively, the temperature sensor may monitor the temperature of the pump cell 55, and the predetermined time Tk may be the time between when the temperature of the pump cell 55 decreases to a predetermined temperature at which the function of the pump cell 55 substantially stops and when such a state has continued for a specific time.

In each of the embodiments, the accumulated value calculating section 70 starts calculating the accumulated value when the reduction process ends. However, the accumulated value calculating may start at any time after the reduction process starts. For example, the accumulated value calculating may start after a predetermined time has elapsed since the reduction process ends. In this case, the accumulated value is calculated for a predetermined period that starts when the predetermined time has elapsed since ending the reduction process. After starting the reduction process and stopping the voltage application to the heater 53 (step S301) or to the pump cell 55 (step S601), calculation of the accumulated value by the accumulated value calculating section 70 may start before the predetermined time Tk has elapsed. In this case, the accumulated value may be calculated for a predetermined period that starts immediately after starting the reduction process.

In each of the embodiments, the accumulation period, in which the accumulated value calculating section 70 accumulates detection values, may be modified. For example, regardless of whether the discharge function of the pump cell 55 has recovered, the time in which a predetermined time has elapsed since the detection function of the sensor cell 56 recovers after starting the reduction process may be set as the accumulation period.

Further, another embodiment may include a temperature sensor that measures the temperature of the pump cell 55, for example. This embodiment starts monitoring the temperature of the pump cell 55 when the stopping section 67 or 91 starts the reduction process, and measures the time between when the reduction process starts and when the temperature of the pump cell 55 reaches a predetermined temperature at which the function of the pump cell 55 substantially stops. Then, the period between when the reduction process ends and when the measured time has elapsed is set as the accumulation period. When the time required to reduce the function of the pump cell 55 is substantially equal to the time required to recover the function of the sensor cell 56, the accumulated value may be calculated accordingly in the period up to when the function of the pump cell 55 is estimated to recover.

In each of the embodiments, the reduction process stops the application of voltage to the heater 53 or to the pump cell 55. Alternatively, the discharge function of the pump cell 55 may be reduced by lowering the applied voltage as compared with the voltage before performing the reduction process.

In each of the embodiments, the configuration of the NOx sensor 40 is not limited to that described above. For example, as long as a signal corresponding to the NOx concentration is output, the configuration of the NOx sensor 40 may be modified. In the NOx sensor 40, the positions of the pump cell 55 and the sensor cell 56 may be exchanged. Specifically, the pump cell 55 may be placed on the side where the wall 43 is located, and the sensor cell 56 may be placed on the side where the diffusion resistive element 42 is located. Further, the pump cell 55 and the sensor cell 56 may be placed at the same position in the exhaust flow direction, so that the pump cathode electrode 55A of the pump cell 55 faces the sensor cathode electrode 56A of the sensor cell 56.

In each of the embodiments, the NOx sensor abnormality detector is used for the NOx sensor 40 placed in the exhaust passage of a diesel engine, but may also be used for a NOx sensor placed in the exhaust passage of a gasoline engine. In this case, power is fed to the controller 60 for a predetermined time after stopping the internal combustion engine, and the abnormality detection control is performed with the exhaust gas caused by scavenging of the internal combustion engine flowing to the NOx sensor. Since such exhaust gas contains a large amount of oxygen, abnormality detection of the NOx sensor is achievable with this configuration in the same manner as the embodiments. Further, abnormality detection control of the NOx sensor may be performed when the combustion state of the gasoline engine is lean.

In each of the embodiments, the controller 60 is not limited to a device that includes a central processing unit and a memory and executes all the above-described processes through software. For example, the controller 60 may include dedicated hardware (an application specific integrated circuit: ASIC) that executes at least part of the various processes. That is, the controller 60 may be circuitry including 1) one or more dedicated hardware circuits such as an ASIC, 2) one or more processors (microcomputers) that operate according to a computer program (software), or 3) a combination thereof.

## Claims

1. A NOx sensor abnormality detector for a NOx sensor (40),
the NOx sensor (40) comprising
a measurement chamber (44), which is located in an exhaust passage (20) of an internal combustion engine and into which exhaust gas is introduced,
a pump cell (55) having a discharge function of discharging oxygen from the measurement chamber (44), and
a sensor cell (56) having a decomposition function of decomposing NOx in the measurement chamber (44) into nitrogen and oxygen and a detection function of detecting an oxygen concentration in the measurement chamber (44),
the NOx sensor (40) is configured to output, as a signal corresponding to a NOx concentration in the exhaust gas, a detection value of oxygen concentration detected by the sensor cell (56) while causing the discharge function of the pump cell (55) to take effect,
the NOx sensor abnormality detector comprising:
a signal receiving section (65) configured to receive the detection value of the sensor cell (56);
a reduction process executing section (66) configured to execute a reduction process that reduces the discharge function of the pump cell (55); and
an abnormality determining section (72) configured to perform an abnormality determination of the NOx sensor (40), wherein
the NOx sensor abnormality detector is configured to execute abnormality detection control in which the abnormality determining section (72) performs abnormality determination of the NOx sensor (40) based on the detection value of the sensor cell (56), which is input to the signal receiving section (65), after the reduction process executing section (66) starts the reduction process.

2. The NOx sensor abnormality detector according to claim 1, further comprising an accumulated value calculating section configured to accumulate detection values of the sensor cell (56), which are input to the signal receiving section (65), during a predetermined accumulation period, which starts after the reduction process starts,
wherein the abnormality determining section (72) is configured to determine that the NOx sensor (40) operates abnormally when an accumulated value calculated by the accumulated value calculating section is less than or equal to a determination value.

3. The NOx sensor abnormality detector according to claim 1 or 2, further comprising an idle determining section (71) configured to determine whether the internal combustion engine is at idle,
wherein the abnormality determining section (72) is configured to execute the abnormality detection control when the idle determining section (71) determines that the internal combustion engine is at idle.
